(19) Europäisches Patentamt
European Patent Office
Office européen des brevets

(11) Veröffentlichungsnummer: **0 277 572**
**A2**

(12) **EUROPÄISCHE PATENTANMELDUNG**

(21) Anmeldenummer: **88101002.9**

(22) Anmeldetag: **25.01.88**

(51) Int. Cl.[4]: **C07D 233/60** , C07D 249/08 , //C07C143/68,C07D317/34, C07C33/26

(30) Priorität: **03.02.87 DE 3703082**

(43) Veröffentlichungstag der Anmeldung: **10.08.88 Patentblatt 88/32**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Kraatz, Udo, Dr.**
**Andreasstrasse 22a**
**D-5090 Leverkusen 1(DE)**

(54) **Verfahren zur Herstellung optisch aktiver 2-Hydroxyethyl-azol-Derivate.**

(57) Nach einem neuen Verfahren sich optisch aktive 2-Hydroxyethyl-azol-Derivate der Formel

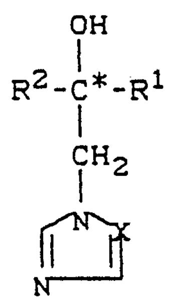

(I)

in welcher
$R^1$, $R^2$ und X die in der Beschreibung angegebene Bedeutung haben,
herstellen, indem man

a) in einer ersten Stufe optisch aktive Diole der Formel

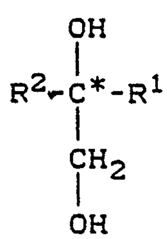

(II)

mit Tosylhalogeniden der Formel

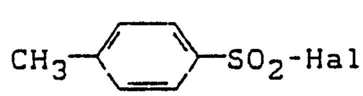

(III)

EP 0 277 572 A2

in welcher
Hal für Chlor oder Brom steht,
in Gegenwart eines Säurebindemittels sowie in Gegenwart eines Verdünnungsmittels umsetzt und

b) in einer zweiten Stufe die so erhaltenen Tosylate der Formel

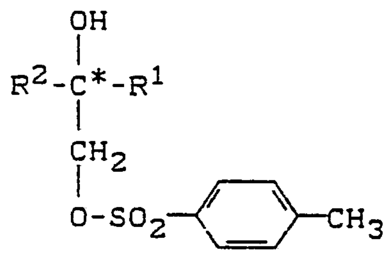

(IV)

mit Azolen der Formel

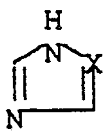

(V)

in Gegenwart eines Säurebindemittels sowie gegebenenfalls in Gegenwart eines Verdünnungsmittels umsetzt.

## Verfahren zur Herstellung optisch aktiver 2-Hydroxyethyl-azol-Derivate

Die vorliegende Anmeldung betrifft ein neues Verfahren zur Herstellung von teilweise bekannten, optisch aktiven 2-Hydroxyethyl-azol-Derivaten, welche fungizide und pflanzenwuchsregulierende Wirksamkeit besitzen.

Es sind bereits Racemate von zahlreichen 2-Hydroxyethyl-azol-Derivaten mit fungiziden und pflanzenwuchsregulierenden Eigenschaften bekannt (vgl. EP-OS 0 040 345, EP-OS 0 052 424 und EP-OS 0 084 834). Die Wirkung dieser Stoffe ist aber vor allem bei niedrigen Aufwandmengen nicht immer befriedigend.

Weiterhin ist bekannt, daß sich optisch aktive 2-Hydroxyethyl-azol-Derivate dadurch herstellen lassen, daß man die zugrunde liegenden Racemate von Oxiranen mit optisch aktiven Sulfonsäuren umsetzt, dann das entstehende Diastereomeren-Gemisch in die reinen Diastereomeren auftrennt und anschließend das jeweils gewünschte Diastereomere mit Azol zur Reaktion bringt (vgl. EP-OS 0 181 529 und DE-OS 3 440 112). Nachteilig an diesem Verfahren ist jedoch, daß die Ausbeuten an der jeweils gewünschten optisch aktiven Komponente relativ niedrig sind, da bedingt durch die Auftrennung des Diastereomeren-Gemisches zumindest 50 % des eingesetzten Materials nicht ausgenutzt werden können.

Es wurde nun gefunden, daß sich optisch aktive 2-Hydroxyethyl-azol-Derivate der Formel

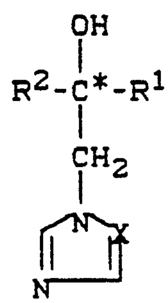

(I)

in welcher
$R^1$ für Alkyl, gegebnenfalls substituiertes Cycloalkyl, gegebenenfalls substituiertes Cycloalkylalkyl oder gegebenenfalls substituiertes Aryl steht,
$R^2$ für Alkyl, Alkinyl, Cycloalkyl, Cycloalkylalkyl, gegebenenfalls substituiertes Benzyl oder für die Reste der Formeln $-CH_2-CH_2-R^3$, $-CH_2-O-R^3$, $-C\equiv C-R^3$ oder $-CH_2-CH=CH-R^4$ steht, wobei
$R^3$ für gegebenenfalls substituiertes Phenyl steht und
$R^4$ für Wasserstoff oder gegebenenfalls substituiertes Phenyl steht,
und
X für ein Stickstoffatom oder eine CH-Gruppe steht,
herstellen lassen, indem man
a) in einer ersten Stufe optisch aktive Diole der Formel

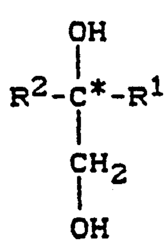

(II)

in welcher
$R^1$ und $R^2$ die oben angegebene Bedeutung haben,
mit Tosylhalogeniden der Formel

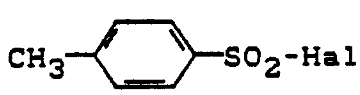

(III)

in welcher
Hal für Chlor oder Brom steht,
in Gegenwart eines Säurebindemittels sowie gegebenenfalls in Gegenwart eines Verdünnungsmittels umsetzt und

b) in einer <u>zweiten Stufe</u> die so erhaltenen Tosylate der Formel

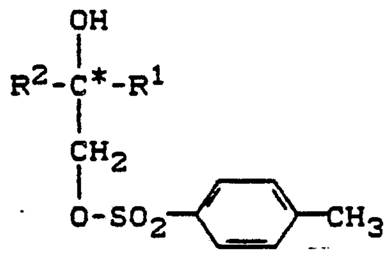

(IV)

in welcher
$R^1$ und $R^2$ die oben angegebene Bedeutung haben,
mit Azolen der Formel

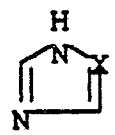

(V)

in welcher
X die oben angegebene Bedeutung hat,
in Gegenwart eines Säurebindemittels sowie gegebenenfalls in Gegenwart eines Verdünnungsmittels umsetzt.

Im vorliegenden Falle sind asymmetrisch substituierte Kohlenstoffatome in den Formelzeichnungen dann durch ein (*) markiert, wenn es sich um optisch aktive Verbindungen handelt. Außer den gekennzeichneten Kohlenstoffatomen können noch weitere asymmetrisch substituierte Kohlenstoffatome enthalten sein.

Der Verlauf des erfindungsgemäßen Verfahrens ist als äußerst überraschend zu bezeichnen. So konnte nicht erwartet werden, daß die als Zwischenprodukte auftretenden Tosylate bei der Reaktion mit Azolen ohne Racemisierung in die optisch aktiven 2-Hydroxyethyl-azol-Derivate der Formel (I) überführt werden können. Unerwartet ist auch, daß sich die gewünschten optischen aktiven Endprodukte nach der erfindungsgemäßen Methode mit hoher Selektivität in sehr guter Ausbeute herstellen lassen.

Des erfindungsgemäße Verfahren zeichnet sich durch eine Reihe von Vorteilen aus. So sind die benötigten Ausgangsstoffe und Reaktionskomponenten leicht zugänglich und auch in größeren Mengen verfügbar. Weiterhin bereiten die einzelnen Umsetzungen und die Aufarbeitung der jeweils anfallenden Reaktionsprodukte keinerlei Schwierigkeiten. Hervorzuheben ist außerdem, daß das gesamte Ausgangsmaterial jeweils zu dem gewünschten Enantiomeren umgesetzt wird. Ein weiterer Vorteil besteht schließlich darin, daß durch den Einsatz des entsprechenden Ausgangsmaterials jeweils sowohl die R-als auch die S-Enantiomeren gleichermaßen gut zugänglich sind.

Verwendet man (2R)-1,2-Diphenyl-propan-2,3-diol und p-Tosylchlorid als Ausgangsstoffe und 1,2,4-Triazol als Reaktionskomponente, so kann der Verlauf des erfindungsgemäßen Verfahrens durch das folgende Formelschema veranschaulicht werden:

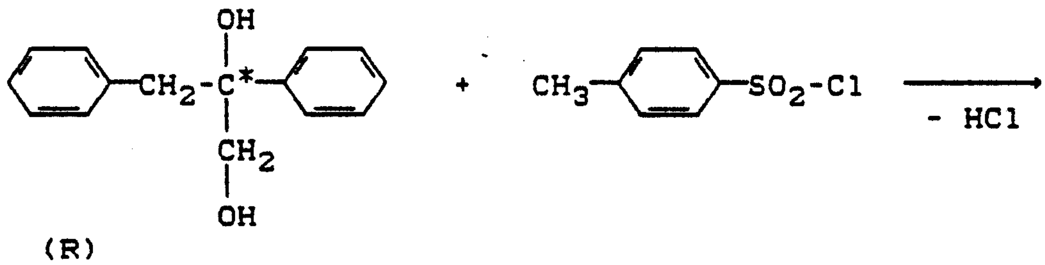

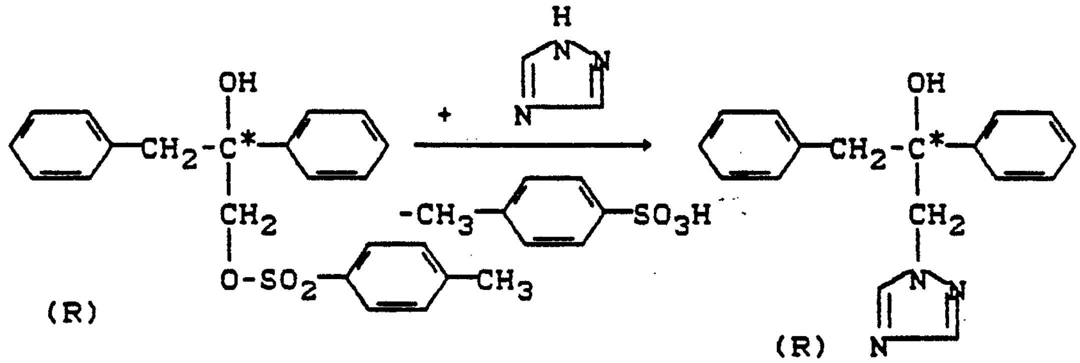

Die bei der Durchführung des erfindungsgemäßen Verfahrens als Ausgangsstoffe benötigten optisch aktiven Diole sind durch die Formel (II) allgemein definiert. Bevorzugt sind diejenigen Stoffe der Formel (II), in denen

$R^1$ für Alkyl mit 1 bis 6 Kohlenstoffatomen, gegebenenfalls einfach bis dreifach, gleichartig oder verschieden durch Alkyl mit 1 bis 4 Kohlenstoffatomen und/oder Halogen substituiertes Cycloalkyl mit 3 bis 8 Kohlenstoffatomen, gegebenenfalls einfach bis dreifach, gleichartig oder verschieden durch Alkyl mit 1 bis 4 Kohlenstoffatomen und/oder Halogen substituiertes Cycloalkylalkyl mit 3 bis 8 Kohlenstoffatomen im Cycloalkylteil und 1 bis 4 Kohlenstoffatomen im Alkylteil oder für gegebenenfalls einfach bis dreifach, gleichartig oder verschieden durch Alkyl mit 1 bis 6 Kohlenstoffatomen, Halogen, Halogenalkyl mit 1 oder 2 Kohlenstoffatomen und 1 bis 5 Halogenatomen, Nitro und/oder Hydroxy substituiertes Phenyl steht und

$R^2$ für geradkettiges oder verzweigtes Alkyl mit 1 bis 8 Kohlenstoffatomen, Alkinyl mit 3 bis 8 Kohlenstoffatomen, Cycloalkyl mit 3 bis 8 Kohlenstoffatomen, Cycloalkylalkyl mit 3 bis 8 Kohlenstoffatomen im Cycloalkylteil und 1 bis 4 Kohlenstoffatomen im Alkylteil, gegebenenfalls im Phenylteil einfach bis dreifach, gleichartig oder verschieden durch Alkyl mit 1 bis 4 Kohlenstoffatomen und/oder Halogen substituiertes Benzyl oder für die Reste der Formeln $-CH_2-CH_2-R^3$, $-CH_2-O-R^3$, $-C{\equiv}C-R^3$ oder $-CH_2-CH=CH-R^4$ steht, wobei

$R^3$ vorzugsweise für gegebenenfalls einfach bis dreifach, gleichartig oder verschieden durch Alkyl mit 1 bis 4 Kohlenstoffatomen, Alkoxy mit 1 bis 4 Kohlenstoffatomen, Halogen und/oder Phenyl substituiertes Phenyl steht und

$R^4$ vorzugsweise für Wasserstoff oder für gegebenenfalls einfach bis dreifach, gleichartig oder verschieden durch Alkyl mit 1 bis 4 Kohlenstoffatomen, Alkoxy mit 1 bis 4 Kohlenstoffatomen, Halogen und/oder Phenyl substituiertes Phenyl steht.

Besonders bevorzugt sind die jenigen R-bzw. S-Enantiomeren von Diolen der Formel (II), in denen

$R^1$ für Alkyl mit 1 bis 4 Kohlenstoffatomen, gegebenenfalls einfach bis dreifach, gleichartig oder verschieden durch Methyl, Ethyl, Fluor, Chlor und/oder Brom substituiertes Cycloalkyl mit 3 bis 6 Kohlenstoffatomen, gegebenenfalls einfach bis dreifach, gleichartig oder verschieden durch Methyl, Ethyl, Fluor, Chlor und/oder Brom substituiertes Cycloalkylalkyl mit 3 bis 6 Kohlenstoffatomen im Cycloalkylteil und 1 bis 3 Kohlenstoffatomen im Alkylteil oder für gegebenenfalls einfach bis dreifach, gleichartig oder verschieden durch Alkyl mit 1 bis 4 Kohlenstoffatomen, Fluor, Chlor, Brom, Trichlormethyl, Trifluormethyl, Nitro und/oder Hydroxy substituiertes Phenyl steht und

$R^2$ für geradkettiges oder verzweigtes Alkyl mit 1 bis 6 Kohlenstoffatomen, Alkinyl mit 3 bis 6 Kohlenstoffatomen, Cycloalkyl mit 3 bis 6 Kohlenstoffatomen, Cycloalkylalkyl mit 3 bis 6 Kohlenstoffatomen im Cycloalkylteil und 1 bis 3 Kohlenstoffatomen im Alkylteil, gegebenenfalls im Phenylteil einfach bis dreifach, gleichartig oder verschieden durch Methyl, Ethyl, Isopropyl, tert.-Butyl, Fluor, Chlor und/oder Brom substituiertes Benzyl oder für die Reste der Formeln $-CH_2-CH_2-R^3$, $-CH_2-O-R^3$, $-C{\equiv}C-R^3$ oder $-CH_2-CH=CH-R^4$ steht, wobei

$R^3$ bevorzugt für gegebenenfalls einfach bis dreifach, gleichartig oder verschieden durch Methyl, Ethyl, Isopropyl, tert.-Butyl, Methoxy, Ethoxy, Fluor, Chlor, Brom und/oder Phenyl substituiertes Phenyl steht und

$R^4$ bevorzugt für Wasserstoff oder für gegebenenfalls einfach bis dreifach, gleichartig oder verschieden

durch Methyl, Ethyl, Isopropyl, tert.-Butyl, Methoxy, Ethoxy, Fluor, Chlor, Brom und/oder Phenyl substituiertes Phenyl steht.

Als Beispiele für optisch aktive Diole der Formel (II) seien genannt:
(2S)-1,2-Diphenyl-propan-2,3-diol,
(4R)-4-Phenyl-pent-1-en-4,5-diol,
(4S)-4-Phenyl-pent-1-en-4,5-diol,
(3R)-1-(4-Chlorphenyl)-3-tert.-butyl-butan-3,4-diol,
(3S)-1-(4-Chlorphenyl)-3-tert.-butyl-butan-3,4-diol,
(2R)-1-(4-Chlorphenoxy)-2-tert.-butyl-propan-2,3-diol.

Die optisch aktiven Diole der Formel (II) sind teilweise bekannt (vgl. Tetrahedron 40, 1313 (1984)). Sie lassen sich herstellen, indem man optisch aktive Dioxolanone der Formel

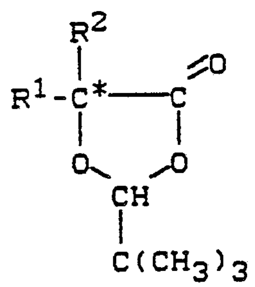

(VI)

in welcher
$R^1$ und $R^2$ die oben angegebene Bedeutung haben,
mit komplexen Hydriden, wie z.B. Lithiumaluminiumhydrid, in Gegenwart eines Verdünnungsmittels, wie z.B. Ether, bei Temperaturen zwischen 0°C und 40°C umsetzt.

Bei der oben angegebenen Reduktion der optisch aktiven Dioxolanone der Formel (VI) erfolgt keine Konfigurationsumkehr am Chiralitätszentrum. Daher entstehen aus den R-Enantiomeren der Formel (VI) die ensprechenden E-Enantiomeren der Formel (II). Ebenso entstehen nach dieser Methode aus den S-Enantiomeren der Formel (VI) die S-Enantiomeren der Formel (II).

Bevorzugte Dioxolanone der Formel (VI) sind die R-bzw. S-Enantiomeren, in denen $R^1$ und $R^2$ diejenigen Bedeutungen haben, die im Zusammenhang mit der Beschreibung der Verbindungen der Formel (II) für diese Reste als bevorzugt genannt wurden.

Als Beispiele für optisch aktive Dioxolanone der Formel (VI) seien genannt:
(2S, 5S)-2-tert.-Butyl-5-benzyl-5-phenyl-1,3-dioxolAn-4-on,
(2R, 5R)-2-tert.-Butyl-5-allyl-5-phenyl-1,3-dioxolan-4-on und
(2S, 5S)-2-tert.-Butyl-5-allyl-5-phenyl-1,3-dioxolan-4-on.

Die optisch aktiven Dioxolanone der Formel (VI) sind teilweise bekannt (vgl. Tetrahedron 40, 1313 (1984)). Sie lassen sich herstellen, indem man optisch aktive Dioxolanone der Formel

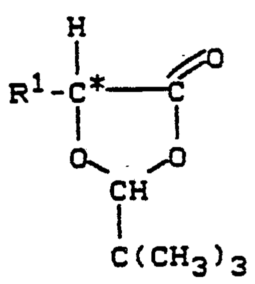

(VII)

in welcher
$R^1$ die oben angegebene Bedeutung hat,
mit Verbindungen der Formel

$R^2$-Y (VIII)

in welcher
$R^2$ die oben angegebene Bedeutung hat und
Y für Chlor, Brom, Iod, Tosylat oder Mesylat steht,
in Gegenwart einer starken Base sowie in Gegenwart eines Katalysators und in Gegenwart eines

Verdünnungsmittels bei Temperaturen zwischen -80°C und +80°C umsetzt.

Die bei dem obigen Verfahren zur Herstellung von optisch aktiven Dioxolanonen der Formel (VI) als Ausgangsstoffe benötigten optisch aktiven Dioxolanone sind durch die Formel (VII) allgemein definiert. In dieser Formel hat $R^1$ vorzugsweise diejenigen Bedeutungen, die bereits im Zusammenhang mit der Beschreibung der Stoffe der Formel (II) vorzugsweise für diesen Rest genannt wurden.

Die optisch aktiven Dioxolanone der Formel (VII) sind teilweise bekannt (vgl. Tetrahedron 40, 1313 (1984)). Sie lassen sich herstellen, indem man optisch aktive Hydroxycarbonsäuren der Formel

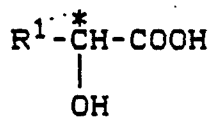

(IX)

in welcher

$R^1$ die oben angegebene Bedeutung hat,

mit Pivalaldehyd der Formel

$(CH_3)_3C\text{-}CHO$ (X)

in Gegenwart eines Verdünnungsmittels, wie z.B. Toluol, und in Gegenwart einer katalytischen Menge einer starken Säure, wie z.B. p-Toluolsulfonsäure, bei Temperaturen zwischen 100°C und 120°C umsetzt.

Die optisch aktiven Hydroxycarbonsäuren der Formel (IX) sind bekannt oder lassen sich nach prinzipiell bekannten Methoden in einfacher Weise herstellen.

Der Pivalaldehyd der Formel (X) ist ebenfalls bekannt.

Die bei dem obigen Verfahren zur Herstellung von optisch aktiven Dioxolanonen der Formel (VI) als Reaktionskomponenten benötigten Verbindungen sind durch die Formel (VIII) allgemein definiert. In dieser Formel hat $R^2$ vorzugsweise diejenigen Bedeutungen, die bereits im Zusammenhang mit der Beschreibung der Stoffe der Formel (II) vorzugsweise für diesen Rest genannt wurden. Y steht für Chlor, Brom, Iod, Tosylat oder Mesylat.

Die Verbindungen der Formel (VIII) sind bekannt oder lassen sich nach prinzipiell bekannten Verfahren in einfacher Weise herstellen.

Als starke Basen kommen bei dem obigen Verfahren zur Herstellung von optisch aktiven Dioxolanone der Formel (VI) alle für derartige Umsetzungen üblichen Deprotonierungsmittel in Betracht. Vorzugsweise verwendbar ist n-Butyl-lithium.

Als Katalysatoren kommen bei dem obigen Verfahren zur Herstellung von optisch aktiven Dioxolanone der Formel (VI) alle für derartige Umsetzungen üblichen Reaktionsbeschleuniger infrage. Vorzugsweise verwendbar sind basische Verbindungen, wie Diisopropylamin und Bis-trimethylsilyl-amin.

Als Verdünnungsmittel können bei dem obigen Verfahren zur Herstellung von optisch aktiven Dioxolanonen der Formel (VI) alle für derartige Umsetzungen üblichen Solventien eingesetzt werden. Vorzugsweise verwendbar sind Ether, wie Tetrahydrofuran.

Die bei der Durchführung des erfindungsgemäßen Verfahrens weiterhin als Ausgangsstoffe benötigten Tosylhalogenide sind durch die Formel (III) definiert. Verbindungen dieser Formel sind p-Tosylchlorid und p-Tosylbromid.

Die Tosylhalogenide der Formel (III) sind ebenso wie die in der zweiten Stufe des erfindungsgemäßen Verfahrens als Reaktionskomponenten benötigten Azole der Formel (IV) bekannte Verbindungen der organischen Chemie.

Als Säurebindemittel kommen bei der Durchführung der ersten Stufe des erfindungsgemäßen Verfahrens alle üblichen Säureakzeptoren in Betracht. Vorzugsweise verwendbar sind Alkalicarbonate, wie Natrium-und Kaliumcarbonat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Als Verdünnungsmittel kommen bei der Durchführung der ersten Stufe des erfindungsgemäßen Verfahrens alle für derartige Umsetzungen üblichen organischen Solventien infrage. Vorzugsweise verwendbar sind polare organische Lösungsmittel, z.B. Nitrile, wie Acetonitril und Propionitril, und ferner aliphatische, aromatische oder heterocyclische Amine, wie Triethylamin, Dimethylanilin, Dimethylbenzylamin, Pyridin, 1,5-Diaza-bicyclo-[4,3,0]-non-5-en, 1,8-Diaza-bicyclo-[5,4,0]-undec-7-en und 1,4-Diaza-bicyclo-[2,2,2]-octan.

Bei der Durchführung der zweiten Stufe des erfindungsgemäßen Verfahrens kommen als Säurebindemittel wiederum alle für derartige Umsetzungen üblichen Säureakzeptoren im Betracht. Vorzugsweise verwendbar sind diejenigen Stoffe, die bereits im Zusammenhang mit der Beschreibung der ersten Stofe des erfindungsgemäßen Verfahrens als Säureakzeptoren ganannt wurden.

Als Verdünnungsmittel kommen bei der Durchführung der zweiten Stufe des erfindungsgemäßen Verfahrens ebenfalls alle für derartige Umsetzung üblichen organischen Lösungsmittel infrage. Vorzugsweise verwendbar sind aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorobenzol und o-Dichlorbenzol, Ether wie Dimethyl- und Dibutylether, Glykoldimethylether und Diglykoldimethylether, Tetrahydrofuran und Dioxan, Ketone wie Aceton, Methylethyl-, Methylisopropyl-und Methyl-isobutyl-keton, Ester wie Essigsäuremethylester und -ethylester, Nitrile wie z.B. Acetonitril und Propionitril und Pyridin.

Die Reaktionstemperaturen können bei der Durchführung des erfindungsgemäßen Verfahrens sowohl in der ersten Stufe als auch in der zweiten Stufe innerhalb eines größeren Bereiches variiert werden. Im allgemeinen arbeitet man in der ersten Stufe bei Temperaturen zwischen 0°C und 100°C, vorzugsweise zwischen 20°C und 60°C. Bei der Durchführung der zweiten Stufe arbeitet man im allgemeinen bei Temperaturen zwischen 0°C und 150°C, vorzugsweise zwischen 20°C und 120°C.

Im allgemeinen arbeitet man bei der Durchführung des erfindungsgemäßen Verfahrens unter Normaldruck. Es ist jedoch auch möglich, sowohl in der ersten als auch in der zweiten Stufe unter erhöhtem oder vermindertem Druck zu arbeiten.

Bei der Durchführung des erfindungsgemäßen Verfahrens setzt man in der ersten Stufe auf 1 Mol an optisch aktivem Diol der Formel (II) im allgemeinen 1 bis 2 Mol an Tosylhalogenid der Formel (III) sowie 1 bis 2 Mol oder auch einen größeren Überschuß an Säurebindemittel ein. In der zweiten Stufe setzt man pro Mol an Tosylat der Formel (IV) im allgemeinen 2 bis 5 Mol an Azol der Formel (V) sowie 2 bis 3 Mol an Säurebindemittel ein. In einzelnen verfährt man in der Weise, daß man zunächst optisch aktives Diol der Formel (II) mit Tosylhalogenid der Formel (III) in Gegenwart von einem Säurebindemittel, das gegebenenfalls auch als Solvens fungieren kann, zur Reaktion bringt. Nach beendeter Umsetzung arbeitet man im allgemeinen in der Weise auf, daß man das Reaktionsgemisch in Eiswasser gibt, das entstehende Gemisch mehrfach mit einem in Wasser wenig löslichen organischen Solvens extrahiert, die vereinigten organischen Phasen nacheinander mit verdünnter, wäßriger Säure und Wasser wäscht und gegebenenfalls nach vorherigem Trocknen unter vermindertem Druck einengt. Das so erhaltene Tosylat der Formel (IV) wird dann mit Azol der Formel (V) in Gegenwart eines Säurebindemittels umgesetzt. Die anschließende Aufarbeitung erfolgt im allgemeinen in der Weise, daß man das Reaktionsgemisch in Wasser gießt, das im allgemeinen in fester Form anfallende Produkt abtrennt und auf chromotographischem Wege oder nach anderen Methoden von noch enthaltenen Verunreinigungen befreit.

In einer besonderen Variante kann das erfindungsgemäße Verfahren auch als Eintopfreaktion durchgeführt werden. Hierbei entfällt die intermediäre Isolierung des Tosylats der Formel (IV).

Die nach dem erfindungsgemäßen Verfahren herstellbaren optisch aktiven 2-Hydroxyethyl-azol-Derivate der Formel (I) besitzen sehr gute fungizide und pflanzenwuchsregu lierende Eigenschaften (vgl. EP-OS 0 040 345, EP-OS 0 052 424 und EP-OS 0 084 834).

Das erfindungsgemäße Verfahren wird durch die folgenden Beispiele veranschaulicht.

Beispiel 1

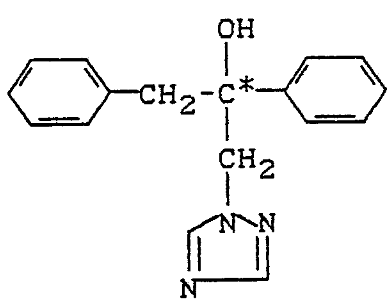

(I-1)

Ein Gemisch aus 87 g (0,23 Mol) (2R)-1,2-Diphenyl-2-hydroxy-3-tosyloxy-propan, 70 g (1 Mol) 1,2,4-Triazol, 70 g (0,5 Mol) Kaliumcarbonat und 800 ml Acetonitril wird unter Rühren 16 Stunden unter Rückfluß erhitzt. Danach gießt man das Reaktionsgemisch in Wasser und saugt das ausgefallene Produkt ab. Zur Abtrennung von noch vorhandenen unerwünschten Nebenprodukten löst man das Produkt in Chloroform und chromatographiert über eine Kieselgel-Säule, wobei als Laufmittel ein Gemisch aus Chloroform/Essigester = 1:1 verwendet wird. Das Eluat wird unter vermindertem Druck eingeengt. Man

erhält auf diese Weise 32,5 g (51 % der Theorie) an (2R)-1,2-Diphenyl-2-hydroxy-3-(1,2,4-triazol-1-yl)-pronan in Form einer Festsubstanz vom Schmelzpunkt 134°C.

$[\alpha]_D^{20}$ = -2,7° (c = 2,75 / $CHCl_3$)

Herstellung von Vorprodukten

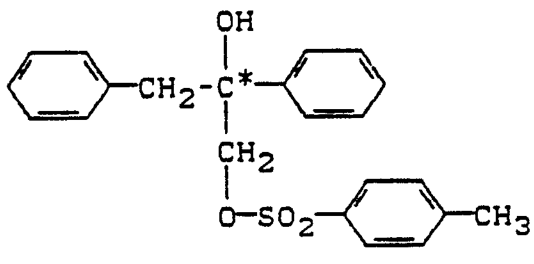

(IV-1)

Zu einer Lösung von 63 g (0,28 Mol) (2R)-1,2-Diphenylpropan-2,3-diol in 600 ml Pyridin werden unter Eiskühlung und unter Rühren 66,2 g (0,35 Mol) p-Tosylchlorid portionweise hinzugeführt. Man läßt das Reaktionsgemisch 16 Stunden bei Raumtemperatur stehen und gießt es dann in Eiswasser. Man extrahiert das entstehende Gemisch mehrfach mit Methylenchlorid, wäscht die vereinigtenorganischen Extrakte mehrfach mit verdünnter wäßriger Salzsäure und danach mit Wasser. Die organische Phase wird unter vermindertem Druck eingeengt. Dabei verbleiben als Rückstand 87,2 g (83 % der Theorie) an (2R)-1,2-Diphenyl-2-hydroxy-3-tosyloxy-propan in Form einer Festsubstanz vom Schmelzpunkt 98°C.

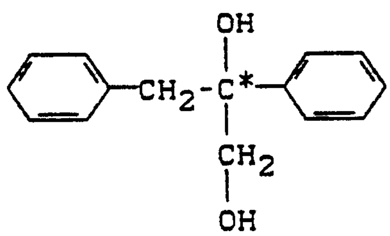

(II-1)

Zu einer Suspension von 15,3 g (0,4 Mol) Lithiumaluminiumhydrid in 700 ml Ether wird unter Rühren eine Lösung von 90 g (0,3 Mol) (2R,4R)-2-tert.-Butyl-5-benzyl-5-phenyl-1,3-dioxolan-4-on in 150 ml Ether so zugetropft, daß die entstehende Lösung schwach unter Rückfluß siedet. Nach beendeter Zugabe wird noch 1 Stunde unter Rückfluß erhitzt. Anschließend wird das Reaktionsgemisch mit Eis gekühlt und langsam mit 20 ml Eiswasser versetzt. Sobald das vorhandene Lithiumaluminiumhydrid hydrolysiert ist, fügt man 500 ml Wasser hinzu und säuert mit verdünnter wäßriger Salzsäure an. Die organische Phase wird abgetrennt und nach mehrmaligem Waschen mit Wasser unter vermindertem Druck eingeengt. Als Rückstand erhält man 63 g (92 % der Theorie) in (2R)-1,2-Diphenyl-propan-2,3-diol in Form eines zunächst öligen Produktes, das nach längerem Stehen durchkristallisiert.
Schmelzpunkt: 62°C.

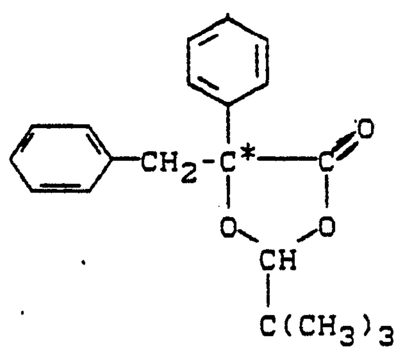

(VI-1)

Zu einer Lösung von 37 g (0,36 Mol) Diisopropylamin in 1 Liter absolutem Tetrahydrofuran tropft man bei -78°C unter Argon 144 ml (0,36 Mol) einer Lösung von n-Butyllithium in n-Hexan. Man rührt noch 15

Minuten bei -78°C nach und tropft dann ebenfalls bei -78°C eine Lösung von 62,1 g (0,3 Mol) (2R,4R)-2-tert.-Butyl-5-phenyl-1,3-di oxolan-5-on in 380 ml absolutem Tetrahydrofuran in das Reaktionsgemisch. Nach 30-minütigem Nachrühren bei -78°C tropft man 60,5 g (0,35 Mol) Benzylbromid bei -78°C hinzu und läßt noch eine Stunde bei dieser Temperatur nachrühren. Anschließend entfernt man das Kühlbad und läßt die Temperatur des Reaktionsgemisches auf 20°C ansteigen. Durch langsame Zugabe von 50 ml Wasser wird hydrolysiert. Danach wird das Reaktionsgemisch unter vermindertem Druck eingeengt. Der verbleibende Rückstand wird mit Methylenchlorid und Wasser versetzt. Die organische Phase wird abgetrennt, zweimal mit Wasser gewaschen unter vermindertem Druck eingeengt. Dabei verbleiben als Rückstand 90 g (97 % der Theorie) an (2R,5R)-2-tert.-Butyl-5-benzyl-5-phenyl-1,3-dioxolan-4-on in Form eines Öles, das durch Behandeln mit Petrolether zur Kristallisation gebracht wird.
Schmelzpunkt: 50°C.

Nach der im Beispiel 1 angegebenen Methode werden auch die in den folgenden Beispielen aufgeführten Stoffe hergestellt.

Beispiel 2

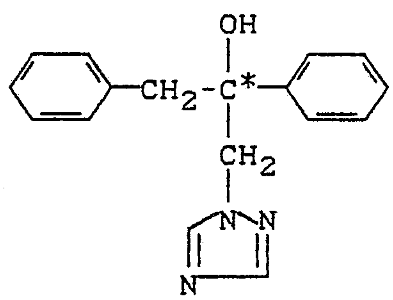

(I-2)

(2S)-1,2-Diphenyl-2-hydroxy-3-(1,2,4-triazol-1-yl)-propan

Fp = 136°C
$[\alpha]_D^{20}$ = +2,8° (c = 0,537 / $CHCl_3$)

Beispiel 3

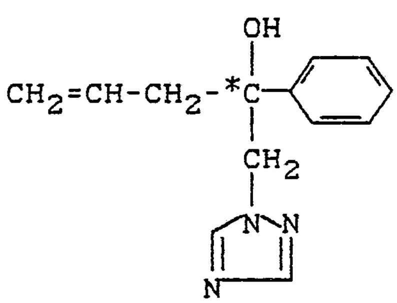

(I-3)

(4S)-4-Hydroxy-4-phenyl-5-(1,2,4-triazol-1-yl)-pent-1-en

Öl; $[\alpha]_D^{20}$ = +29,6° (c - 0,692/$CHCl_3$)

Beispiel 4

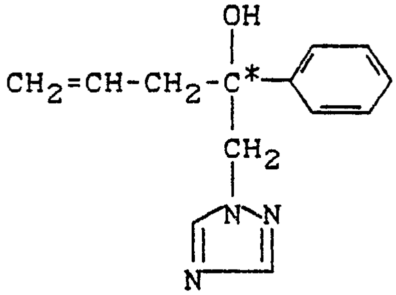

(I-4)

(4R)-4-Hydroxy-4-phenyl-5-(1,2,4-triazol-1-yl)-pent-1-en

Öl; $[\alpha]_D^{20}$ = -28,6° (c = 0,765 / $CHCl_3$)

**Ansprüche**

1. Verfahren zur Herstellung von optisch aktiven 2-Hydroxyethyl-azol-Derivaten der Formel

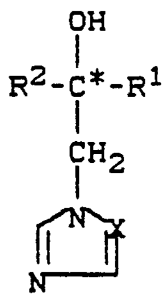

(I)

in welcher
$R^1$ für Alkyl, gegebenenfalls substituiertes Cycloalkyl, gegebenenfalls substituiertes Cycloalkylalkyl oder gegebenenfalls substituiertes Aryl steht,
$R^2$ für Alkyl, Alkinyl, Cycloalkyl, Cycloalkylalkyl, gegebenenfalls substituiertes Benzyl oder für die Reste der Formeln $-CH_2-CH_2-R^3$, $-CH_2-O-R^3$, $-C\equiv C-R^3$ oder $-CH_2-CH=CH-R^4$ steht, wobei
$R^3$ für gegebenenfalls substituiertes Phenyl steht und
$R^4$ für Wasserstoff oder gegebenenfalls substituiertes Phenyl steht,
und
X für ein Stickstoffatom oder eine CH-Gruppe steht,
dadurch gekennzeichnet, daß man a) in einer ersten Stufe optisch aktive Diole der Formel

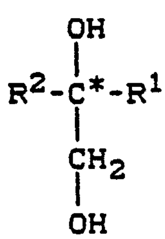

(II)

in welcher
$R^1$ und $R^2$ die oben angegebene Bedeutung haben,
mit Tosylhalogeniden der Formel

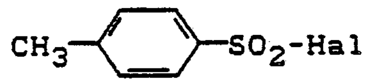 (III)

in welcher
Hal für Chlor oder Brom steht,
in Gegenwart eines Säurebindemittels sowie gegebenenfalls in Gegenwart eines Verdünnungsmittels umsetzt und
b) in einer zweiten Stufe die so erhaltenen Tosylate der Formel

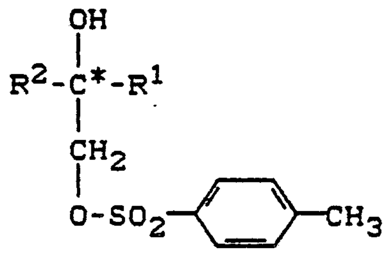 (IV)

in welcher
$R^1$ und $R^2$ die oben angegebene Bedeutung haben,
mit Azolen der Formel

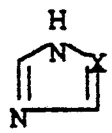 (V)

in welcher
X die oben angegebene Bedeutung hat,
in Gegenwart eines Säurebindemittels sowie gegebenenfalls in Gegenwart eines Verdünnungsmittels umsetzt.

2. Verfahren gemäß Anspruch 1, dadurch gekennzeichnet, daß man optisch aktive Diole der Formel (II) einsetzt, in denen
$R^1$ für Alkyl mit 1 bis 6 Kohlenstoffatomen, gegebenenfalls einfach bis dreifach, gleichartig oder verschieden durch Alkyl mit 1 bis 4 Kohlenstoffatomen und/oder Halogen substituiertes Cycloalkyl mit 3 bis 8 Kohlenstoffatomen, gegebenenfalls einfach bis dreifach, gleichartig oder verschieden durch Alkyl mit 1 bis 4 Kohlenstoffatomen und/oder Halogen substituiertes Cycloalkylalkyl mit 3 bis 8 Kohlenstoffatomen im Cycloalkylteil und 1 bis 4 Kohlenstoffatomen im Alkylteil oder für gegebenenfalls einfach bis dreifach, gleichartig oder verschieden durch Alkyl mit 1 bis 6 Kohlenstoffatomen, Halogen, Halogenalkyl mit 1 oder 2 Kohlenstoffatomen und 1 bis 5 Halogenatomen, Nitro und/oder Hydroxy substituiertes Phenyl steht und
$R^2$ für geradkettiges oder verzweigtes Alkyl mit 1 bis 8 Kohlenstoffatomen, Alkinyl mit 3 bis 8 Kohlenstoffatomen, Cycloalkyl mit 3 bis 8 Kohlenstoffatomen, Cycloalkylalkyl mit 3 bis 8 Kohlenstoffatomen im Cycloalkylteil und 1 bis 4 Kohlenstoffatomen im Alkylteil, gegebenenfalls im Phenylteil einfach bis dreifach, gleichartig oder verschieden durch Alkyl mit 1 bis 4 Kohlenstoffatomen und/oder Halogen substituiertes Benzyl oder für die Reste der Formeln $-CH_2-CH_2-R^3$, $-CH_2-O-R^3$, $-C{\equiv}C-R^3$ oder $-CH_2-CH=CH-R^4$ steht, wobei
$R^3$ vorzugsweise für gegebenenfalls einfach bis dreifach, gleichartig oder verschieden durch Alkyl mit 1 bis 4 Kohlenstoffatomen, Alkoxy mit 1 bis 4 Kohlenstoffatomen, Halogen und/oder Phenyl substituiertes Phenyl steht und
$R^4$ vorzugsweise für Wasserstoff oder für gegebenenfalls einfach bis dreifach, gleichartig oder verschieden durch Alkyl mit 1 bis 4 Kohlenstoffatomen, Alkoxy mit 1 bis 4 Kohlenstoffatomen, Halogen und/oder Phenyl substituiertes Phenyl steht.

3. Verfahren gemäß Anspruch 1, dadurch gekennzeichnet, daß man optisch aktive Diole der Formel (II) einsetzt, in denen
$R^1$ für Alkyl mit 1 bis 4 Kohlenstoffatomen, gegebenenfalls einfach bis dreifach, gleichartig oder verschieden durch Methyl, Ethyl, Fluor, Chlor und/oder Brom substituiertes Cycloalkyl mit 3 bis 6 Kohlenstoffatomen, gegebenenfalls einfach bis dreifach, gleichartig oder verschieden durch Methyl, Ethyl, Fluor, Chlor und/oder Brom substituiertes Cycloalkylalkyl mit 3 bis 6 Kohlenstoffatomen im Cycloalkylteil und 1 bis 3 Kohlenstoffatomen im Alkylteil oder für gegebenenfalls einfach bis dreifach, gleichartig oder verschieden durch Alkyl mit 1 bis 4 Kohlenstoffatomen, Fluor, Chlor, Brom, Trichlormethyl, Trifluormethyl, Nitro und/oder Hydroxy substituiertes Phenyl steht und

$R^2$ geradkettiges oder verzweigtes Alkyl mit 1 bis 6 Kohlenstoffatomen, Alkinyl mit 3 bis 6 Kohlenstoffatomen, Cycloalkyl mit 3 bis 6 Kohlenstoffatomen, Cycloalkylalkyl mit 3 bis 6 Kohlenstoffatomen im Cycloalkylteil und 1 bis 3 Kohlenstoffatomen im Alkylteil, gegebenenfalls im Phenylteil einfach bis dreifach, gleichartig oder verschieden durch Methyl, Ethyl, Isopropyl, tert.-Butyl, Fluor, Chlor und/oder Brom substituiertes Benzyl oder für die Reste der Formeln

$-CH_2-CH_2-R^3$, $-CH_2-O-R^3$, $-C\equiv C-R^3$ oder $-CH_2-CH=CH-R^4$ steht, wobei

$R^3$ für gegebenenfalls einfach bis dreifach, gleichartig oder verschieden durch Methyl, Ethyl, Isopropyl, tert.-Butyl, Methoxy, Ethoxy, Fluor, Chlor, Brom und/oder Phenyl substituiertes Phenyl steht und

$R^4$ für Wasserstoff oder für gegebenenfalls einfach bis dreifach, gleichartig oder verschieden durch Methyl, Ethyl, Isopropyl, tert.-Butyl, Methoxy, Ethoxy, Fluor, Chlor, Brom und/oder Phenyl substituiertes Phenyl steht.

4. Verfahren gemäß Anspruch 1, dadurch gekennzeichnet, daß man als Ausgangsstoff der Formel (III) Tosylchlorid einsetzt.

5. Verfahren gemäß Anspruch 1, dadurch gekennzeichnet, daß man als Reaktionskomponente der Formel (V) 1,2,4-Triazol einsetzt.

6. Verfahren gemäß Anspruch 1, dadurch gekennzeichnet, daß man als Ausgangsstoffe (2R)-1,2-Diphenyl-propan-2,3-diol und p-Tosylchlorid einsetzt und 1,2,4-Triazol als Reaktionskomponente einsetzt.

7. Verfahren gemäß Anspruch 1, dadurch gekennzeichnet, daß man als Ausgangsstoffe (2S)-1,2,-Diphenyl-propan-2,3-diol und p-Tosylchlorid einsetzt und 1,2,4-Triazol als Reaktionskomponente einsetzt.

8. Verfahren gemäß Anspruch 1, dadurch gekennzeichnet, daß man als Ausgangsstoffe (4S)-4-Phenyl-pent-1-en-4,5-diol und p-Tosylchlorid einsetzt und 1,2,4-Triazol als Reaktionskomponente einsetzt.

9. Verfahren gemäß Anspruch 1, dadurch gekennzeichnet, daß man als Ausgangsstoffe (4R)-4-Phenyl-pent-1-en-4,5-diol und p-Tosylchlorid einsetzt und 1,2,4-Triazol als Reaktionskomponente einsetzt.

10. Verfahren gemäß Anspruch 1, dadurch gekennzeichnet, daß man die Umsetzung in der ersten Stufe bei Temperaturen zwischen 0°C und 100°C und die Umsetzung in der zweiten Stufe bei Temperaturen zwischen 0°C und 150°C durchführt.